# EUROPÄISCHE PATENTSCHRIFT

(11) **EP 1 497 662 B1**
(45) Veröffentlichungstag und Bekanntmachung des Hinweises auf die Patenterteilung: **13.09.2006**
(21) Anmeldenummer: 03725036.2
(22) Anmeldetag: 15.04.2003
(51) Int. Cl.: G01N 33/68, G01N 33/569, G01N 33/573

(54) **VERWENDUNGEN DER CARBAMOYLPHOSPHAT SYNTHETASE 1 (CPS 1) UND IHRER FRAGMENTE FÜR DIE DIAGNOSE VON SEPSIS**
USES OF THE CARBAMOYL PHOSPHATE SYNTHESIS 1 (CPS 1) AND THE FRAGMENTS THEREOF FOR THE DIAGNOSIS OF SEPSIS
UTILISATION DE CARBAMOYL-PHOSPHATE SYNTHETASE 1 (CPS1) ET DE SES FRAGMENTS DANS LE DIAGNOSTIC DE MALADIES DE SEPSIES

(30) Priorität: 19.04.2002 EP 02008841
(43) Veröffentlichungstag der Anmeldung: 19.01.2005
(73) Patentinhaber: B.R.A.H.M.S Aktiengesellschaft, 16761 Hennigsdorf (DE)
(72) Erfinder: BERGMANN, Andreas, 12351 Berlin (DE); STRUCK, Joachim, 12161 Berlin (DE); ÜHLEIN, Monika, 10407 Berlin (DE)
(74) Vertreter: Andrae, Steffen
(86) Internationale Anmeldenummer: PCT/EP2003/003939
(87) Internationale Veröffentlichungsnummer: WO 2003/089933

(56) Entgegenhaltungen:
- WO-A-00/73322
- ARDAWI M S M: "HEPATIC GLUTAMINE METABOLISM IN THE SEPTIC RAT" CLINICAL SCIENCE (LONDON), Bd. 82, Nr. 6, 1992, Seiten 709-716, XP008008364 ISSN: 0143-5221
- TABUCHI SHOKO ET AL: "Regulation of genes for inducible nitric oxide synthase and urea cycle enzymes in rat liver in endotoxin shock." BIOCHEMICAL AND BIOPHYSICAL RESEARCH COMMUNICATIONS, Bd. 268, Nr. 1, 5. Februar 2000 (2000-02-05), Seiten 221-224, XP001113123 ISSN: 0006-291X in der Anmeldung erwähnt
- SCHIMKE T: "Adaptive Characteristics of Urea Cycle Enzymes in the Rat" JOURNAL OF BIOLOGICAL CHEMISTRY, Bd. 237, 1962, Seiten 459-468, XP008008974
- SCHOUW NIELSEN S ET AL: "ACUTE SYSTEMIC AND LOCAL INFLAMMATION DECREASES HEPATIC EXPRESSION OF UREA CYCLE ENZYMES" JOURNAL OF HEPATOLOGY, MUNKSGAARD INTERNATIONAL PUBLISHERS, COPENHAGEN, DK, Bd. 32, Nr. SUPPL 2, 29. Mai 2000 (2000-05-29), Seite 161 XP001159742 ISSN: 0168-8278
- OZAKI M ET AL: "ENZYME-LINKED IMMUNOSORBENT ASSAY OF CARBAMOYLPHOSPHATE SYNTHETASE I: PLASMA ENZYME IN RAT EXPERIMENTAL HEPATITIS AND ITS CLEARANCE" ENZYME PROTEIN, KARGER, BASEL, CH, Bd. 48, Nr. 4, 1994, Seiten 213-221, XP008018977 ISSN: 1019-6773
- TYGSTRUP N ET AL: "EXPRESSION OF LIVER FUNCTIONS FOLLOWING SUB-LETHAL AND NON-LETHAL DOSES OF ALLYL ALCOHOL AND ACETAMINOPHEN IN THE RAT" JOURNAL OF HEPATOLOGY, MUNKSGAARD INTERNATIONAL PUBLISHERS, COPENHAGEN, DK, Bd. 27, Nr. 1, Juli 1997 (1997-07), Seiten 156-162, XP001159744 ISSN: 0168-8278
- YIN L ET AL: "PARTICIPATION OF DIFFERENT CELL TYPES IN THE RESTITUTIVE RESPONSE OF THE RAT LIVER TO PERIPORTAL INJURY INDUCED BY ALLYL ALCOHOL" JOURNAL OF HEPATOLOGY, MUNKSGAARD INTERNATIONAL PUBLISHERS, COPENHAGEN, DK, Bd. 31, Nr. 3, September 1999 (1999-09), Seiten 497-507, XP001159743 ISSN: 0168-8278
- DATABASE BIOSIS [Online] BIOSCIENCES INFORMATION SERVICE, PHILADELPHIA, PA, US; 1989 SZONDY Z ET AL: "EFFECT OF POLYAMINES ON THE CARBAMOYLPHOSPHATE SYNTHETASE ACTIVITY OF CAD PROTEIN" Database accession no. PREV199089003267 XP002249138 & ACTA BIOCHIMICA ET BIOPHYSICA HUNGARICA, Bd. 24, Nr. 1-2, 1989, Seiten 107-118, ISSN: 0237-6261

## Beschreibung

Die vorliegende Erfindung betrifft Verwendungen des Enzyms Carbamoylphosphat Synthetase 1 (E.C. 6.3.4.16, nachfolgend stets abgekürzt CPS 1) und neuer Fragmente davon für die medizinische Diagnostik von Sepsis. Sie beruht auf dem erstmaligen Nachweis des Auftretens von Fragmenten der CPS 1 in Lebergewebe von Primaten, bei denen experimentell durch Toxinverabreichunge eine Sepsis bzw. systemische Entzündung erzeugt worden war, sowie auf dem anschließenden Nachweis stark erhöhter Konzentrationen von CPS 1 in der Zirkulation von Sepsispatienten.

Die vorliegende Erfindung hat ihren Ursprung in intensiven Forschungsarbeiten der Anmelderin im Zusammenhang mit weiteren Verbesserungen der Diagnose und Therapie von Entzündungen und Infektionen, insbesondere von Entzündungen infektiöser Ätiologie und Sepsis.

Als Entzündungen (Inflammationen) werden ganz allgemein bestimmte physiologische Reaktionen eines Organismus auf verschiedenartige äußere Einwirkungen wie z.B. Verletzungen, Verbrennungen, Allergene, Infektionen durch Mikroorganismen wie Bakterien und Pilze und Viren, auf Fremdgewebe, die Abstoßungsreaktionen auslösen, oder auf bestimmte entzündungsauslösende endogene Zustände des Körpers, z.B. bei Autoimmunerkrankungen und Krebs, bezeichnet. Entzündungen können als harmlose, lokal begrenzte Reaktionen des Körpers auftreten, sind jedoch auch typische Merkmale zahlreicher ernster chronischer und akuter Erkrankungen von einzelnen Geweben, Organen, Organ- und Gewebsteilen.

Lokale Entzündungen sind dabei meist Teil der gesunden Immunreaktion des Körpers auf schädliche Einwirkungen, und damit Teil des lebenserhaltenden Abwehrmechanismus des Organismus. Wenn Entzündungen jedoch Teil einer fehlgeleiteten Reaktion des Körpers auf bestimmte endogene Vorgänge wie z.B. bei Autoimmunerkrankungen sind und/oder chronischer Natur sind, oder wenn sie systemische Ausmaße erreichen, wie beim systemischen Inflammationssyndrom (Systemic Inflammatory Response Syndrome, SIRS) oder bei einer auf infektiöse Ursachen zurückzuführenden schweren Sepsis, geraten die für Entzündungsreaktionen typischen physiologischen Vorgänge außer Kontrolle und werden zum eigentlichen, häufig lebensbedrohlichen Krankheitsgeschehen.

Es ist heute bekannt, dass die Entstehung und der Verlauf von entzündlichen Prozessen von einer beträchtlichen Anzahl von Substanzen, die überwiegend proteinischer bzw. peptidischer Natur sind, gesteuert werden bzw. von einem mehr oder weniger zeitlich begrenzten Auftreten bestimmter Biomoleküle begleitet sind. Zu den an Entzündungsreaktionen beteiligten endogenen Substanzen gehören insbesondere solche, die zu den Cytokinen, Mediatoren, vasoaktiven Substanzen, Akutphasenproteinen und/oder hormonellen Regulatoren gezählt werden können. Die Entzündungsreaktion stellt eine komplexe physiologische Reaktion dar, an der sowohl das Entzündungsgeschehen aktivierende endogene Substanzen (z.B. TNF-α) als auch desaktivierende Substanzen (z.B. Interleukin-10) beteiligt sind.

Bei systemischen Entzündungen wie im Falle einer Sepsis bzw. des septischen Schocks weiten sich die entzündungsspezifischen Reaktionskaskaden unkontrolliert auf den gesamten Körper aus und werden dabei, im Sinne einer überschießenden Immunantwort, lebensbedrohlich. Zu den gegenwärtigen Kenntnissen über das Auftreten und die mögliche Rolle einzelner Gruppen endogener entzündungsspezifischer Substanzen wird beispielsweise verwiesen auf A.Beishuizen, et al., "Endogenous Mediators in Sepsis and Septic Shock", Advances in Clinical Chemistry, Vol.33, 1999, 55-131; und C.Gabay, et al., "Acute Phase Proteins and Other Systemic Responses to Inflammation", The New England Journal of Medicine, Vol.340, No.6, 1999, 448-454. Da sich das Verständnis von Sepsis und verwandten systemischen entzündlichen Erkrankungen, und damit auch die anerkannten Definitionen, in den letzten Jahren gewandelt haben, wird außerdem verwiesen auf K.Reinhart, et al., "Sepsis und septischer Schock", in: Intensivmedizin, Georg Thieme Verlag, Stuttgart.New York, 2001, 756-760; wo eine moderne Definition des Sepsis-Begriffes gegeben wird. Im Rahmen der vorliegenden Anmeldung werden die Begriffe Sepsis bzw. entzündliche Erkrankungen in Anlehnung an die Definitionen verwendet, wie sie den genannten drei Literaturstellen entnommen werden können.

Während wenigstens im europäischen Raum die durch eine positive Blutkultur nachweisbare systemische bakterielle Infektion lange den Sepsisbegriff prägte, wird die Sepsis heute in erster Linie als systemische Entzündung verstanden, die infektiöse Ursachen hat, als Krankheitsgeschehen jedoch große Ähnlichkeiten mit systemischen Entzündungen aufweist, die durch andere Ursachen ausgelöst werden. Dem genannten Wandel des Sepsis-Verständnisses entsprechen Veränderungen der diagnostischen Ansätze. So wurde der direkte Nachweis bakterieller Erreger durch komplexe Überwachungen physiologischer Parameter und in jüngerer Zeit insbesondere auch durch den Nachweis bestimmter am Sepsisgeschehen bzw. am Entzündungsgeschehen beteiligter endogener Substanzen, d.h. spezifischer "Biomarker", ersetzt bzw. ergänzt.

Von der großen Zahl von Mediatoren und Akutphasenproteinen, von denen man weiß, dass sie an einem Entzündungsgeschehen beteiligt sind, eignen sich dabei für diagnostische Zwecke insbesondere solche, deren Auftreten sehr spezifisch für entzündliche Erkrankungen bzw. bestimmte Phasen entzündlicher Erkrankungen ist, deren Konzentrationen sich drastisch und diagnostisch signifikant verändern und die außerdem die für Routinebestimmungen erforderlichen Stabilitäten aufweisen und hohe Konzentrationswerte erreichen. Für diagnostische Zwecke steht dabei die zuverlässige Korrelation von Krankheitsgeschehen (Entzündung, Sepsis) mit dem jeweiligen Biomarker im Vordergrund, ohne dass dessen Rolle in der komplexen Kaskade der am Entzündungsgeschehen beteiligten endogenen Substanzen bekannt sein muss.

Eine derartige als Sepsis-Biomarker besonders geeignete endogene Substanz ist Procalcitonin. Procalcitonin ist ein Prohormon, dessen Serum-Konzentrationen unter den Bedingungen einer systemischen Entzündung infektiöser Ätiologie (Sepsis) sehr hohe Werte erreichen, während es bei Gesunden so gut wie nicht nachweisbar ist. Hohe Werte an Procalcitonin werden außerdem in einem relativ frühen Stadium einer Sepsis erreicht, so dass sich die Bestimmung von Procalcitonin auch zur Früherkennung einer Sepsis und zur frühen Unterscheidung einer infektiös bedingten Sepsis von schweren Entzündungen, die auf anderen Ursachen beruhen, eignet. Die Bestimmung von Procalcitonin als Sepsismarker ist Gegenstand der Veröffentlichung M.Assicot, et al., "High serum procalcitonin concentrations in patients with sepsis and infection", The Lancet, vol.341, No.8844, 1993, 515-518; und der Patente DE 42 27 454 C2 bzw. EP 0 656 121 B1 bzw. US 5,639,617. Auf die genannten Patente und in der genannten Veröffentlichung angeführte frühere Literaturstellen wird zur Ergänzung der vorliegenden Beschreibung ausdrücklich Bezug genommen.

Die Verfügbarkeit des Sepsismarkers Procalcitonin hat der Sepsisforschung starke Impulse gegeben, und es werden gegenwärtig intensive Anstrengungen unternommen, weitere Biomarker zu finden, die die Procalcitonin-Bestimmung ergänzen können und/oder zusätzliche Informationen für Zwecke der Feindiagnostik bzw. Differentialdiagnostik zu liefern vermögen. Erschwert wird die Suche nach potentiellen neuen Sepsis-Biomarkern allerdings dadurch, dass häufig noch sehr wenig oder nichts über die genaue Funktion bzw. über die genauen Gründe für das Auftreten bestimmter endogener Substanzen, die am Entzündungs- oder Sepsisgeschehen beteiligt sind, bekannt ist.

Die Ergebnisse der experimentellen Überprüfung eines fruchtbaren rein hypothetischen Ansatzes zur Ermittlung weiterer potentieller Sepsismarker finden sich in DE 198 47 690 A1 bzw. WO 00/22439. Dort wird gezeigt, dass bei Sepsis nicht nur die Konzentration des Prohormons Procalcitonin erhöht ist, sondern auch für andere Substanzen, die Prohormon-Immunreaktivität aufweisen, signifikant erhöhte Konzentrationen beobachtet werden können. Während das beschriebene Phänomen gut dokumentiert ist, sind die Ursachen für den Anstieg der Konzentrationen der verschiedenen entsprechenden Substanzen bei Sepsis weiterhin weitgehend ungeklärt.

In der vorliegenden Anmeldung wird ein Ergebnis eines anderen fruchtbaren, rein experimentellen Ansatzes für die Suche nach weiteren entzündungs- bzw. sepsisspezifischen Biomolekülen berichtet. Auch diese experimentellen Untersuchungen nehmen ihren Ausgang bei der Bestimmung von Procalcitin im Zusammenhang mit systemischen entzündlichen Reaktionen infektiöser Ätiologie. So war sehr früh beobachtet worden, dass bei Sepsis das Procalcitonin offensichtlich nicht auf die gleiche Weise gebildet wird, wie dann, wenn es Vorläufer für das Hormon Calcitonin ist. So wurden hohe Procalcitoninspiegel auch bei Patienten beobachtet, denen die Schilddrüse entfernt worden war. Deshalb kann die Schilddrüse nicht dasjenige Organ sein, in dem Procalcitonin bei Sepsis gebildet bzw. ausgeschüttet wird. In den Veröffentlichungen H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", Sepsis 1998; 2:243-253; werden die Ergebnissen von experimentellen Untersuchungen berichtet, die der Klärung der Bildung von Procalcitonin bei Sepsis dienen sollten. In den genannten Arbeiten wird durch Endotoxinverabreichung an Primaten (Paviane) eine künstliche Sepsis erzeugt, und es wird bestimmt, bei welchen experimentell erzeugten Zuständen die höchsten Procalcitoninkonzentrationen im Blut erreicht werden. Eine Weiterentwicklung des in den genannten Arbeiten beschriebene Versuchstiermodells dient im Rahmen der vorliegenden Anmeldung dazu, neue endogene sepsisspezifische Biomarker von peptischer bzw. proteinischer Natur zu ermitteln, deren Auftreten für Sepsis oder bestimmte Formen von Sepsis charakteristisch ist und die daher eine spezifische Sepsisdiagnose ermöglichen. Das Primatenmodell wurde dabei aufgrund der sehr großen Ähnlichkeit der Physiologie von Primaten und Menschen und der hohen Kreuzreaktivität mit vielen therapeutischen und diagnostischen humanen Reagenzien gewählt.

Da die bei Entzündungen gebildeten endogenen Substanzen Teil der komplexen Reaktionskaskade des Körpers sind, sind derartige Substanzen außerdem nicht nur von diagnostischem Interesse, sondern es wird gegenwärtig auch mit erheblichem Aufwand versucht, durch Beeinflussung der Entstehung und/oder der Konzentration einzelner derartiger Substanzen therapeutisch in das Entzündungsgeschehen einzugreifen, um die zum Beispiel bei Sepsis beobachtete systemische Ausbreitung der Entzündung möglichst frühzeitig zu stoppen. In diesem Sinne sind nachweislich am Entzündungsgeschehen beteiligte endogene Substanzen auch als potentielle therapeutische Targets anzusehen. Trotz der bisherigen eher enttäuschenden Ergebnisse derartiger therapeutischer Ansätze besteht weiterhin ein hohes Interesse daran, bisher im entsprechenden Zusammenhang nicht beschriebene, möglichst entzündungs- bzw. sepsisspezifische endogene Biomoleküle zu identifizieren, die auch als therapeutische Targets neue Erfolgsaussichten für die therapeutische Sepsiskontrolle eröffnen.

Die vorliegenden Erfindung beruht darauf, dass sich in Primaten und Menschen bei Sepsis in der Zirkulation deutlich erhöhte Konzentrationen des Enzyms Carbamoylphosphat Synthetase (CPS 1), sowie Fragmente davon, nachweisen lassen, und zwar im Unterschied zu unbehandelten Kontrollindividuen bzw. Gesunden, bei denen diese nicht gefunden werden, was CPS 1 für die Sepsisdiagnosik geeignet macht.

Diagnostische in vitro Verfahren, die aufgrund des erstmals nachgewiesenen Auftretens von CPS 1 und ihrer Fragmente bei der experimentellen Simulation von Sepsis sowie des Nachweises deutlich erhöhter Konzentrationen von CPS 1-Immunreaktivität in Seren von Septikern zugänglich werden, werden in den Ansprüchen 1 bis 10 beansprucht.

Wie nachfolgend im experimentellen Teil noch näher ausgeführt wird, war Ausgangspunkt der Erfindung die Feststellung, dass nach experimenteller Auslösung einer künstlichen Sepsis in Pavianen durch Endotoxinverabreichung (LPS aus Salmonella Typhimurium) und 2D-gelektrophoretischer Aufarbeitung von Lebergewebe der behandelten Tiere eine Gruppe von nur bei den behandelten Tieren identifizierbaren benachbarten Proteinspots gefunden werden konnte. Die den Spots entsprechenden proteinischen Produkte mit (gelektrophoretisch bestimmten) molaren Massen von ca. 68 kDa, 69 kDa und 70 kDa ± 3 kDa wurden aus dem Elektrophoresegel isoliert, massenspektrometrisch untersucht und als lösliche Fragmente von CPS 1 identifiziert.

Anschließend wurde unter Verwendung eines Immunoassays, der die genannten Fragmente erkannte, festgestellt, dass in der Zirkulation von Sepsispatienten in stark erhöhten Konzentrationen Bestandteile mit der Immunreaktivität dieser Fragmente gefunden werden, wobei sich diese Bestandteile bei einer genaueren Identifizierung (u.a. Abtrennung und Molekulargewichtsbestimmung) wenigstens überwiegend als das vollständige, oder wenigstens weitgehend vollständige, Enzym CPS 1 erwiesen.

Bei der massenspektrometrischen Analyse von drei aus dem Gel isolierten Proteinspots, die als solche eine relativ geringe Intensität aufwiesen, durch Tandem-Massenspektrometrie wurden aus allen drei Proteinspots kurze, teilweise identische Teilpeptide ("Tags") identifiziert, die sich in identischer Form in der Sequenz der humanen CPS 1 (SEQ ID NO:6) wiederfanden, wobei die konkret identifizierten Peptide Aminosäuresequenzen aus dem N-terminalen Bereich der CPS 1 Aminosäuren bis zu Position 624 des CPS 1 (SEQ ID NO:6) umfaßten.

Aufgrund der Identität der identifizierten massenspektrometrischen Fragmente mit Teilsequenzen aus dem N-terminalen Teil der CPS 1 ist die Identifizierung der untersuchten Proteinspotss als CPS 1-Fragmente nach anerkannten Kriterien als eindeutig anzusehen.

Die Identifizierung der nur nach Sepsis- bzw. Entzündungsauslösung in dem Pavian-Lebergewebe gefundenen Proteine als Fragmente aus den N-terminalen Teil der CPS 1 ist von hohem wissenschaftlichen, diagnostischen und therapeutischen Interesse.

Die anschließende Feststellung, dass in der Zirkulation von menschlichen Sepsispatienten stark erhöhte Konzentrationen einer oder ggf. mehrerer Spezies mit der Immunreaktivität der identifizierten CPS 1-Fragmente beobachtet werden, die sich jedoch als das vollständige, oder wenigstens im wesentlichen vollständige, Enzym CPS 1 erwiesen, das ggf. auch einer besonderen solubilisierten Form vorliegen kann, erhöhte den Wert der beschriebenen ersten Befunde noch erheblich.

Für die medizinische Diagnostik spielten CPS 1 bzw. CPS 1-Fragmente bisher noch keine praktische Rolle. Das Enzym CPS 1 ((E.C. 6.3.4.16) selbst ist jedoch seit langem gut bekannt. Es katalysiert die Umwandlung von Ammoniak, Bicarbonat und 2 ATP unter Bildung von Carbamoylphosphat im ersten Schritt des Harnstoffzyklus. Es spielt dabei auch eine Rolle bei der Biosynthese von Arginin, das seinerseits ein Substrat für die Biosynthese von NO, z.B. bei einem Endotoxin-Schock, ist (vgl. Shoko Tabuchi et al., Regulation of Genes for Inducible Nitric Oxide Synthase and Urea Cycle Enzymes in Rat Liver in Endotoxin Shock, Biochemical and Biophysical Research Communications 268, 221-224 (2000)). CPS 1 ist von dem cytosolischen Enzym CPS 2 (E.C. 6.3.5.5.) zu unterscheiden, das ebenfalls eine Rolle im Harnstoffzyklus spielt, jedoch das Substrat Glutamin verarbeitet. Es ist bekannt, dass CPS 1 in Mitochondrien lokalisiert ist und im Lebergewebe in dieser Form in großen Mengen (es macht von 2-6% des Leber-Gesamtproteins aus) vorkommt. Seine Aminosäuresequenz (SEQ ID NO:6) und genetische Lokalisierung ist seit langem bekannt (vgl. Haraguchi Y. et al, Cloning and sequence of a cDNA encoding human carbamyl phosphate synthetase I: molecular analysis of hyperammonemia, Gene 1991, Nov. 1; 107 (2):-335-340). Zu seiner physiologischen Rolle kann verwiesen werden auf Reviewartikel wie z.B. H.M.Holder et al., Carbamoyl phosphate synthetase: an amazing biochemical odyssey from substrate to product, CMLS, Cell.Mol.Life Sci. 56 (1999) 507-522 und die darin referierte Literatur, sowie die Einleitung der Veröffentlichung Mikiko Ozaki et al., Enzyme-Linked Immunosorbent Assay of Carbamoylphosphate Synthetase I: Plasma Enzyme in Rat Experimental Hepatitis and Its Clearance, Enzyme Protein 1994, 95:48:213-221.

In einer Arbeit von Ardawi M S M in: Clinical Science (1992) 82, 709-716 wird die Veränderung der Aktivität von Leberenzymen bei septischen Ratten untersucht. Dabei werden einerseits Konzentrationsveränderungen von Metaboliten, die von den Leberenzymen gebildet werden, im Blut der Ratten untersucht. Die Aktivität der Enzyme des Harnstoffzyklus wie CPS 1 wurde in Lebergewebe untersucht. Das Auftreten von CPS 1 bzw. eine CPS 1-Bestimmung in der Zirkulation werden nicht beschrieben.

Gemäß Shoko Tabuchi et al., loc.cit. wird in Rattenlebern bei einem künstlichen Endotoxin-Schock (LPS) keine Erhöhung des Enzyms (Proteins) beobachtet. Gemäß Li Yin et al., Participation of different cell types in the restitutive response of the rat liver to periportal injury induced by allyl alcohol, Journal of Hepatology 1999, 31:497-507, läßt sich bei einer Leberschädigung durch Allylakohol bei histologischen Untersuchungen nach drei Tagen in allen Hepatocyten eine Steigerung der CPS 1-Expression beobachten.

Es wurde ferner festgestellt, dass sich im Rattenmodell bei einer durch Verabreichung von Galactosamin experimentell erzeugten akuten Hepatitis im Rattenplasma eine stark erhöhte immunologische CPS 1-Aktivität findet (nachgewiesen mit einem ELISA mit anti-Ratten CPS 1 IgG aus Kaninchen), und zwar 24-48 h nach der Behandlung mit dem Hepatitis-auslösenden Galactosamin. Im Rattenplasma wurden während der akuten Hepatitis auch zunehmend CPS 1-Fragmente mit molaren Massen von ca. 140 und 125 kDa ohne sonstige nähere Charakterisierung (Sequenzzuordnung) erkennbar, während bei einer begleitenden Immunoblotting-Analyse in humanen Autopsie-Proben keine CPS 1-Fragmente mit CPS 1-Immunreaktivität beobachtet werden konnten (Mikiko Ozaki et al., loc. cit.).

Eine Eignung von im wesentlichen vollständiger CPS 1 und von löslichen CPS 1-Fragmenten, insbesondere von Fragmenten mit molaren Massen von 68-70 kDa ± 3 kDa aus dem N-terminalen Teil der CPS 1, als Biomarker für die Diagnose von Entzündungen und Sepsis bei Menschen, der in humanem Serum oder Plasma bestimmt werden kann, läßt sich aus den Literaturbefunden nicht ableiten.

Aufgrund der nachgewiesenen verstärkten Bildung der humanen CPS 1 bei Sepsis, und von Fragmenten von CPS 1 bei Pavianen mit einer experimentell erzeugten Sepsis, und zwar im Gegensatz zu unbehandelten bzw. gesunden Patienten bzw. Tieren, in deren Zirkulation bzw. Lebergewebe trotz identischer Aufarbeitung und Lagerung keine derartigen Fragmente nachweisbar waren, eignen sich CPS 1 und ihre Fragmente für diagnostische Zwecke. Werden für den Nachweis nach an sich bekannten immundiagnostischen Verfahren CPS 1 und ihre Fragmente als Reagenzien oder zur Erzeugung bestimmter spezifischer Antikörper benötigt, können die Fragment nach Verfahren, die inzwischen zum Stand der Technik gehören, synthetisch oder gentechnologisch als Rekombinationsprodukte hergestellt werden.

Ferner können die benötigten CPS 1-Fragmente nach bekannten Verfahren des modernen Standes der Technik auch zur Erzeugung spezifischer polyklonaler oder monoklonaler Antikörper verwendet werden, die als Hilfsmittel für die diagnostische Bestimmung der erfindungsgemäßen Peptide und/oder auch als potentielle therapeutische Mittel geeignet sind. Die Erzeugung geeigneter monoklonaler oder polyklonaler Antikörper gegen bekannte Peptid-Teilsequenzen gehört heute zum allgemeinen Stand der Technik.

Bei der Bestimmung von CPS 1 bzw. CPS 1-Fragmenten in Patientenseren kann grundsätzlich so vorgegangen werden, wie das z.B. für die selektive Procalcitoninbestimmung beschrieben ist in P.P.Ghillani, et al., "Monoclonal antipeptide antibodies as tools to dissect closely related gene products", The Journal of Immunology, vol. 141, No.9, 1988, 3156-3163; und P.P.Ghillani, et al., "Identification and Measurement of Calcitonin Precursors in Serum of Patients with Malignant Diseases", Cancer research, vol.49, No.23, 1989, 6845-6851; wobei ausdrücklich ergänzend auch auf die dort beschriebenen Immunisierungstechniken verwiesen wird, die eine Möglichkeit für die Gewinnung von monoklonalen Antikörpern auch gegen Teilsequenzen der CPS 1 darstellen. Variationen der beschriebenen Techniken und/oder weitere Immunisierungstechniken kann der Fachmann einschlägigen Standardwerken und Veröffentlichungen entnehmen und sinngemäß anwenden. Ein bevorzugter Immunoassay zur Bestimmung von CPS 1 in humanen biologischen Flüssigkeiten, insbesondere humanem Serum oder Plasma, wird nachfolgend, zusammen mit damit gewonnenen Messergebnissen und der näheren Charakterisierung des nachgewiesenen Analyten, im experimentellen Teil beschrieben.

Als Verwendung im Sinne der vorliegenden Anmeldung ist auch eine Verwendung von CPS 1 bzw. löslichen CPS 1-Fragmenten als Bestandteil (Reagens) eines Assaykits anzusehen, oder eine Verwendung zur Erzeugung von Assaykomponenten, z.B. poly- oder monoklonalen Antikörpern, die z.B. in immobilisierter und/oder markierter Form in der Regel ebenfalls in Assaykits vorgesehen sind.

Dabei ist auch ausdrücklich die Erzeugung von CPS 1-Antikörpern unter Anwendung von Techniken der direkten genetischen Immunisierung mit DNA zu erwähnen. Es liegt im Rahmen der vorliegenden Erfindung, zur Immunisierung z.B. eine cDNA von CPS 1 oder der gewünschten CPS 1-Fragmente zu verwenden, da es sich in der Vergangenheit gezeigt hat, dass durch derartige Immunisierungstechniken das Spektrum der gewinnbaren Antikörper erweitert werden kann.

Es ist zusätzlich ausdrücklich darauf hinzuweisen, dass es bei der erfindungsgemäßen Bestimmung von CPS 1 oder CPS 1-Fragmenten aus dem N-terminalen Teil der CPS 1-Sequenz je nach Assaydesign auch dazu kommen kann, dass ggf. gleichzeitig in der biologischen Flüssigkeit vorhandene andere, z.B. längere lösliche CPS 1-Bruchstücke, die diese Fragmente enthalten, oder auch in löslicher Form vorliegende Formen der vollständigen CPS 1 (die normalerweise in den Mitochondrien lokalisiert ist) bestimmt oder mitbestimmt werden. Auch derartige Verfahren sind, im Sinne der vorliegenden Erfindung, als erfindungsgemäße Verfahren zur Bestimmung von CPS 1 bzw. CPS 1-Fragmenten anzusehen.

CPS 1 gemäß SEQ ID NO:6 oder lösliche Formen davon oder lösliche Teilpeptide davon, z.B. solche, die eine der Teilsequenzen von SEQ ID NO:1 bis SEQ ID NO:5 und/oder andere Teilsequenzen aus dem N-Terminus von CPS 1 enthalten oder daraus bestehen, können somit aufgrund der vorliegenden Ergebnisse als spezifische Markerpeptide (Biomarker) zum diagnostischen Nachweis und zur Verlaufskontrolle von Entzündungen und Infektionen (insbesondere, wie Procalcitonin, auch von systemischen Infektionen vom Sepsistyp) dienen.

Die CPS 1-Bestimmung in der Zirkulation kann für diagnostische Zwecke u.U. auch indirekt als Bestimmung einer Enzymaktivität, die der CPS 1-Aktivität oder der Restaktivität der CPS 1-Fragmente entspricht, erfolgen.

Es ist ferner möglich, die Bestimmung von CPS 1 und/oder CPS 1-Fragmenten als Prognosemarker und Marker für die Überwachung des Krankheitsverlaufs von Entzündungen, insbesondere systemischen Entzündungen, und Sepsis im Rahmen einer Kombinationsmessung mit anderen Markern durchzuführen.

Neben einer Kombination mit einer Procalcitoninmessung kommt insbesondere eine Kombination der Messung von CPS 1 mit der Bestimmung anderer Marker für Sepsis und systemische Entzündungen in Betracht, und zwar insbesondere mit CA 19-9, CA 125, S100B oder an der Regulierung von Entzündungen beteiligten S100A-Proteinen, oder mit der Bestimmung der in den älteren, nachfolgend genannten unveröffentlichten deutschen Patentanmeldungen der Anmelderin beschriebenen neuartigen Sepsismarker Inflammin (DE 101 19 804.3) und CHP (DE 101 31 922.3), des Proteins LASP-1 und/oder mit der Bestimmung von löslichen Cytokeratinfragmenten, insbesondere der neu autgefundenen löslichen Cytokeratin-1-Fragmente (sCY1F;; DE 101 30 985.6) sowie der bekannten Tumormarker CYFRA-21 oder TPS und/oder eines oder mehrerer der o.g. Prohormone. Auch eine gleichzeitige Bestimmung des bekannten Entzündungsparameters C-reaktives Protein (CRP) kann vorgesehen sein. Aufgrund der in dieser und den verwandten Anmeldungen der Anmelderin beschriebenen neuen Ergebnisse ist für die Sepsis-Feindiagnose außerdem generell eine Kombination mit Messungen von bekannten oder noch aufzufindenden Biomolekülen in Betracht zu ziehen, die als gewebe- bzw. organspezifische Entzündungsmarker geeignet sind.

Die eigentliche CPS 1-Bestimmung kann auf irgendeine geeignete, an sich bekannte Weise erfolgen, wobei Immunoassays eines geeigneten Assaydesigns bevorzugt sind.

Bei einer bevorzugten Ausführungsform wird die immundiagnostische Bestimmung als heterogener Sandwich-Immunoassay durchgeführt, bei dem einer der Antikörper an eine beliebige Festphase, beispielsweise die Wände beschichteter Teströhrchen (z.B. aus Polystyrol; "Coated Tubes"; CT) oder an Mikrotiterplatten, zum Beispiel aus Polystyrol, oder an Partikel, beispielsweise Magnetpartikel immobilisiert ist, während der andere Antikörper einen Rest trägt, der ein direkt nachweisbares Label darstellt oder eine selektive Verknüpfung mit einem Label ermöglicht und der Detektion der gebildeten Sandwich-Strukturen dient. Auch eine zeitlich verzögerte bzw. nachträgliche Immobilisierung unter Verwendung geeigneter Festphasen ist möglich.

Grundsätzlich können alle in Assays der beschriebenen Art verwendbaren Markierungstechniken angewandt werden, zu denen Markierungen mit Radioisotopen, Enzymen, Fluoreszenz-, Chemolumineszenz- oder Biolumineszenz-Labeln und direkt optisch detektierbaren Farbmarkierungen, wie beispielsweise Goldatomen und Farbstoffteilchen, wie sie insbesondere für sog. Point-of-Care (POC) oder Schnelltests verwendet werden, gehören. Die beiden Antikörper können auch im Falle heterogener Sandwich-Immunoassays Teile eines Nachweissystems der nachfolgend im Zusammenhang mit homogenen Assays beschriebenen Art aufweisen.

Es liegt somit im Rahmen der vorliegenden Erfindung, das erfindungsgemäße Verfahren auch als Schnelltest auszugestalten.

Das erfindungsgemäße Verfahren kann ferner als homogenes Verfahren ausgestaltet werden, bei dem die aus den beiden Antikörpern und dem nachzuweisenden CPS 1 gebildeten Sandwichkomplexe in der flüssigen Phase suspendiert bleiben. In einem solchen Fall ist es bevorzugt, beide Antikörper mit Teilen eines Nachweissystems zu markieren, das dann, wenn beide Antikörper in einen einzigen Sandwich integriert werden, eine Signalerzeugung oder Signalauslösung ermöglicht. Derartige Techniken sind insbesondere als Fluoreszenzverstärkungs- oder Fluoreszenzlöschungs-Nachweisverfahren ausgestaltbar. Ein besonderes bevorzugtes derartiges Verfahren betrifft die Verwendung von paarweise einzusetzenden Nachweisreagenzien, wie sie beispielsweise beschrieben sind in US-A-4 822 733, EP-B1-180 492 oder EP-B1-539 477 und dem darin zitierten Stand der Technik. Sie ermöglichen eine Messung, die selektiv nur Reaktionsprodukte erfaßt, die beide Markierungskomponenten in einem einzigen Immunkomplex enthalten, direkt in der Reaktionsmischung. Als Beispiel ist auf die unter.den Marken TRACE® (Time Resolved Amplified Cryptate Emission) bzw. KRYPTOR® angebotene Technologie zu verweisen, die die Lehren der o.g. Anmeldungen umsetzt.

Der Inhalt der genannten älteren Anmeldungen der Anmelderin ist durch die ausdrückliche Bezugnahme auf diese Anmeldungen als Teil der Offenbarung der vorliegenden Anmeldung anzusehen.

Nachfolgend wird die Auffindung und Identifizierung der CPS 1-Fragmente, sowie die Bestimmung von Substanzen mit der Immunreaktivität dieser Fragmente in der menschlichen Zirkulation, die sich anschließend als das wenigstens im wesentlichen vollständige Enzym CPS 1 bzw. eine lösliche Form davon erwiesen, in näheren Einzelheiten geschildert, wobei auf das beigefügte Sequenzprotokoll bezug genommen wird. Die Figuren zeigen:

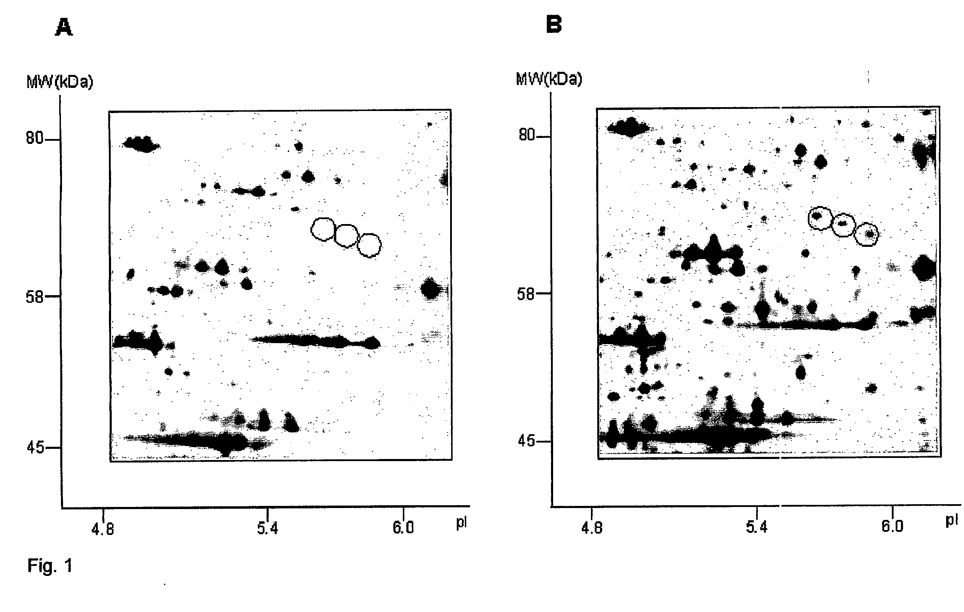

- Fig. 1: Ansichten von 2D-Elektrophoresegelen, die einen Vergleich der Spotmuster von cytoplasmatischen Leberzellproteinen eines gesunden Pavians (A) mit den Leberzellproteinen eines Pavians 5h nach einer durch LPS-Verabreichung induzierten Sepsis (B) ermöglichen. Der Pfeil zeigt die Positionen der drei erfindungsgemäßen sepsisspezifischen Produkte (CPS 1-Fragmente) an, die in Darstellung (B) durch einen Kreis hervorgehoben sind.

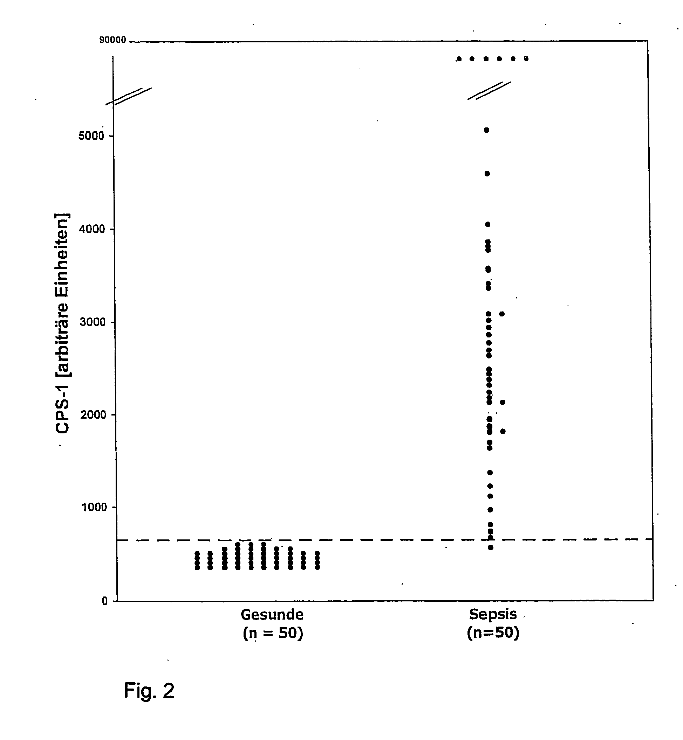

- Fig. 2: die Ergebnisse der Messung der CPS 1-Immunreaktivität in Plasmen von gesunden Normalpersonen und Patienten mit Sepsis mit einem im experimentellen Teil genauer beschreibenen Immunoassay, wobei die gestrichelte Linie die untere Nachwesgrenze des Tests anzeigt.

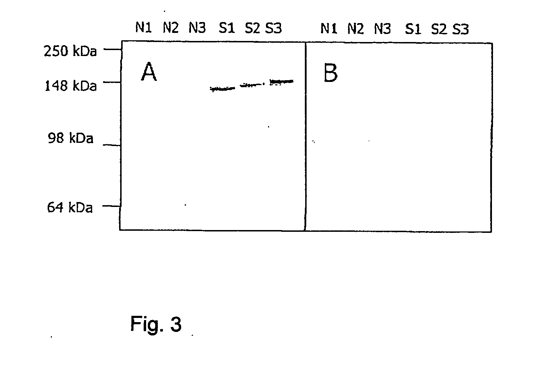

- Fig.3: Western-Blot-Banden von Plasmaproben unter Verwendung von anti-CPS Antiseren. Aufgetragen wurden (Panel A) Proben von Normalpersonen (N1-N3) und Sepsispatienten (S1-S3). Zur Detektion von CPS 1 wurde ein Gemisch von Antiseren gegen zwei definierte CPS 1-Epitope (Pos. 184-199 und 245-257 der CPS 1 gemäß SEQ ID NO:6) eingesetzt. Die Spezifität der Reaktion wurde geprüft, indem in einem zweiten Ansatz (Panel B) die Antiseren mit einem Überschuß der Peptide vorinkubiert wurden, die zur Immunisierung bzw. Gewinnung der Antiseren eingesetzt worden waren. Die Positionen der Molekulargewichtsmarker sind angezeigt.

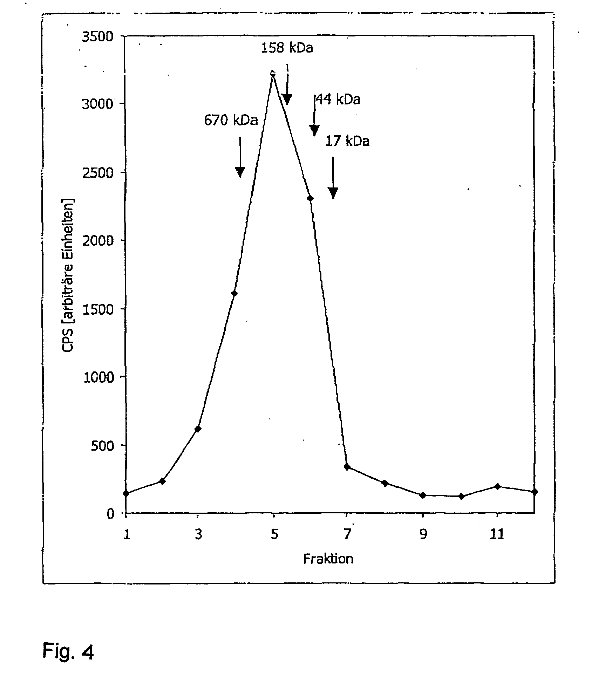

- Fig. 4: ein CPS 1-Immunchromatogramm einer Gelfiltrationschromatographie von Sepsisplasma. 100 µl eines Sepsisplasmas wurden über eine Bio-Sil SEC-400HPLC-Säule chromatographiert. Es wurden Fraktionen ä 1 ml gesammelt, und die CPS 1-Immunreaktivität der einzelnen Fraktionen wurde gemessen. Positionen von Größenstandards, die in einem getrennten Lauf chromatographiert wurden, sind angegeben.

### EXPERIMENTELLER TEIL

### 1. Infektionssimulation durch Endotoxinverabreichung im Tiermodell (Paviane).

In Anlehnung an die mit Pavianen durchgeführten Versuche zur Stimulierung der Procalcitonin-Ausschüttung durch Endotoxininjektionen (vgl.H.Redl, et al., "Procalcitonin release patterns in a baboon model of trauma and sepsis: Relationship to cytokines and neopterin", Crit Care Med 2000, Vol.28. No.11, 3659-3663; H.Redl, et al., "Non-Human Primate Models of Sepsis", in: Sepsis 1998; 2:243-253) wurden Pavianen (männlich, ca. 2 Jahre alt, 27 bis 29 kg schwer) jeweils 100 µg LPS (Lipopolysaccharid aus Salmonella Typhimurium, Bezugsquelle: Sigma) pro kg Körpergewicht intravenös verabreicht. 5 bis 5,5 h nach der Injektion wurden die Tiere durch intravenöse Verabreichung von 10 ml Doletal getötet. Innerhalb von 60 min nach ihrem Exitus wurden sämtliche Organe/Gewebe präpariert und durch Einfrieren in flüssigem Stickstoff stabilisiert.

Bei der weiteren Verarbeitung wurden Proben der einzelnen tiefgefrorenen Gewebe (1g) unter Stickstoffkühlung mit 1,5 ml Puffer A (50mM Tris/HCl, pH 7,1, 100mM KCl, 20% Glycerol) versetzt und in einem Porzellanmörser zu einem Mehl pulverisiert (vgl. J.Klose, "Fractionated Extraction of Total Tissue Proteins from Mouse and Human for 2-D Electrophoresis", in: Methods in Molecular Biology, Vol.112: 2-D Proteome Analysis Protocols, Humana Press Inc., Totowa, NJ). Nach einer anschließenden 1-stündigen Zentrifugation bei 100.000 g und +4°C wurde der erhaltene Überstand gewonnen und bis zur weiteren Verarbeitung bei -80°C gelagert.

Weil Versuche mit den wie oben gewonnenen Proben ergeben hatten, dass die größten Procalcitoninmengen in Lebergewebe behandelter Tiere gefunden wird, wurde für die Suche nach neuen sepsisspezifischen Biomarkern mit Proteinextrakten aus der Pavianleber gearbeitet.

### 2. Proteomanalyse unter Verwendung cytoplasmatischer Leberzellproteine von Pavianen.

Cytoplasmatische Leberzellproteinextrakte von einerseits gesunden Pavianen (Kontrolle) und andererseits Pavianen, denen LPS injiziert worden war, wurden im Rahmen einer Proteomanalyse verwendet. Bei der einleitenden analytischen 2D-Gelelektrophorese wurde Leberextrakt, 100 µg Protein enthaltend, auf 9M Harnstoff, 70 mM DTT, 2% Ampholyt pH 2-4 eingestellt und dann mittels analytischer 2D-Gelelektrophorese aufgetrennt, wie in J.Klose, et al., "Two-dimensional electrophoresis of proteins: An updated protocol and implications for a functional analysis of the genome", Electrophoresis 1995, 16, 1034-1059; beschrieben ist. Die Sichtbarmachung der Proteine im 2D-Elektrophoresegel erfolgte mittels Silverstaining (vgl. J.Heukeshoven, et al., "Improved silver staining procedure for fast staining in Phast-System Development Unit. I. Staining of sodium dodecyl gels", Electrophoresis 1988, 9, 28-32).

Zur Auswertung wurden die Proteinspotmuster der Proben unbehandelter Tieren mit den Proteinspotmustern verglichen, die aus Lebergewebeproben behandelter Tiere resultierten. Substanzen, die bei keiner Kontrollprobe, aber bei allen behandelten Tieren zusätzlich auftraten, wurden für weitere analytische Untersuchungen selektiert. Fig. 1 zeigt einen Vergleich der 2D-Elektrophoresegele für eine Kontrollprobe (A) und eine Probe eines behandelten Tieres (B), wobei drei zusätzliche Proteinspots in (B) mit molaren Massen von ca. 68 kDa, 69 kDA und 70 kDa (± 3 kDa) und isoelektrischen Punkten von ca. 6,0, 5,8 bzw. 5,6 durch einen Pfeil und einen Kreis hervorgehoben sind.

Die im Proteinspotmuster der analytischen 2D-Gelelektrophorese identifizierten neuen spezifischen Proteine wurden dann anschließend mittels präparativer 2D-Gelelektrophorese unter Einsatz von 350 µg Protein präpariert (vgl. wiederum (10). Bei der präparativen 2D-Gelelektrophorese erfolgte die Färbung mittels Coomassie Brilliant Blue G250 (vgl. V.Neuhoff, et al., "Improved staining of proteins in polyacrylamide gels including isoelectric focusing gels with clear background at nanogram sensitivity using Coomassie Brilliant Blue G-250 and R-250", Electrophoresis 1988, 9, 255-262).

Die für die weitere Analyse vorselektierten Proteinspots wurden aus dem Gel ausgeschnitten.

Die Proteinspots wurden jeweils unter Anwendung der Methode, die in A.Otto, et al., "Identification of human myocardial proteins separated by two-dimensional electrophoresis using an effective sample preparation for mass spectrometry", Electrophoresis 1996, 17, 1643-1650; beschrieben ist, trypsinverdaut und anschließend massenspektroskopisch analysiert und zwar unter Anwendung massenspektrometrischer Untersuchungen, wie sie z.B. in G.Neubauer, et al., "Mass spectrometry and EST-database searching allows characterization of the multi-protein spliceosome complex", in: nature genetics vol. 20, 1998, 46-50; J.Lingner, et al., "Reverse Transcriptase Motifs in the Catalytic Subunit of Telomerase", in: Science, Vol.276, 1997, 561-567; M.Mann, et al., "Use of mass spectrometry-derived data to annotate nucleotide and protein sequence databases", in: TRENDS in Biochemical Sciences, Vol.26, 1, 2001, 54-61; beschrieben und diskutiert werden.

Dabei wurden Fragmente des Trypsinverdaus aller drei Proteinspots nach einer ESI (ElectroSprayIonisierung) auch einer Tandem-Massenspektrometrie unterzogen. Es wurde ein Q-TOF-Massenspektrometer mit einer sog. nanoflow-Z-Spray-Ionenquelle der Firma Micromass, UK, verwendet. Dabei wurde entsprechend der Arbeitsanleitung des Geräteherstellers gearbeitet.

### 3. Identifizierung von CPS 1-Fragmenten

Wie in den Figuren 1(A) und 1(B) gezeigt ist, finden sich in Leberzellextrakten von Pavianen, denen eine LPS-Injektion verabreicht worden war, u.a. drei neue Proteinspots, für die aufgrund der Gelektrophoresedaten im Vergleich mit Markersubstanzen mit bekanntem Molekulargewicht Molekulargewichte von ca. 68 kDa, 69 kDa und 70 kDa (± 3 kDa) abgeschätzt wurden, während aus der relativen Position der Proteine aus der ersten Dimension zugehörige isoelektrische Punkte von etwa 6,0, 5,8 bzw. 5,6 ermittelt wurden, d.h. isoelektrische Punkte im Bereich von ca. 5,5 bis 6,1.

Diese Proteine wurden wie oben erläutert massenspektrometrisch analysiert.

Aus den "Mutterspektren" der drei trypsinverdauten Poteine wurden jeweils einzelne Fragmente ("Tags") durch Tandem-Massenspektroskopie identifiziert. Die für diese Fragmente erhaltenen Massenspektren konnten auf an sich bekannte Weise rechnerisch ausgewertet werden und lieferten die folgenden Ergebnisse (massenspektrometrisch ist keine Unterscheidung zwischen den Aminosäuren Leucin (L) und Isoleucin (I) sowie den Aminosäuren Lysin (K) und Glutamin (Q) möglich; die nachfolgenden Sequenzen berücksichtigen daher bereits die Zuordnung zum bekannten Spektrum der vollständigen CPS 1 gemäß SEQ ID NO:6):

| Proteinspot bei 70 kDa (± 3 kDa): | | |
|---|---|---|
| Fragment 70/1: | GQNQPVLNITN | (SEQ ID NO:1) |
| Fragment 70/2: | NQPVLNI | (SEQ ID NO:2) |
| Fragment 70/3: | AQTAHIVLEDGTK | (SEQ ID NO:3) |

| Proteinspot bei 69 kDa (± 3 kDa): | | |
|---|---|---|
| Fragment 69/1: | GQNQPVLNITN | (SEQ ID NO:1) |
| Fragment 69/2: | TAHI | (SEQ ID NO:4) |

| Proteinspot bei 68 kDa (± 3 kDa): | | |
|---|---|---|
| Fragment 68/1: | NQPVLNI | (SEQ ID NO:2) |
| Fragment 68/2: | AFAMTNQILVEK | (SEQ ID NO:5). |

Die obigen Teilsequenzen gemäß SEQ ID NO:1 bis SEQ ID NO:5 konnten als Teilsequenzen der unter NiceProt View of SWISS PROT: P31327 zu findenden Sequenz der humanen CPS 1 mit einer Aminosäurekette einer Länge von 1500 Aminosäuren und einer zugehörigen rechnerischen molaren Masse (ohne Berücksichtigung evtl. posttranslationaler Modifizierungen) von 164,939 kDa (SEQ ID NO:6) identifiziert werden. Es ergab sich die folgenden Zuordnung der Teilpeptide:

| | |
|---|---|
| SEQ ID NO:1 | Aminosäuren 317 - 327 |
| SEQ ID NO:2 | Aminosäuren 319 - 325 |
| SEQ ID NO:3 | Aminosäuren 43 - 55 |
| SEQ ID NO:4 | Aminosäuren 45 - 48 |
| SEQ ID NO:5 | Aminosäuren 613 - 624 |

Die gefundenen Aminosäuren überspannen einen Abschnitt von Aminosäure 43 bis Aminosäure 624, d.h. einen wesentlichen Teil des aminoterminalen Teils der CPS 1.

Es ist ergänzend darauf hinzuweisen, dass sich die gefundenen Sequenzen nicht dem verwandten cytosolischen Enzym CPS 2 zuordnen ließen.

### 4. CPS 1-Immunreaktivitäts-Bestimmungen in humanen Plasmen von gesunden Normalpersonen und Sepsispatienten

### 4.1 Material und Methoden

### 4.1.1. Peptidsynthesen

Abgeleitet von der bekannten Aminosäuresequenz des humanen CPS 1 wurden zwei Bereiche ausgewählt (Pos. 184-199: Peptidbereich 1; SEQ ID NO:7; Pos. 245-257: Peptidbereich 2; SEQ ID NO:8). Jeweils ergänzt um einen N-terminalen Cystein-Rest wurden beide Bereiche nach Standardverfahren als lösliche Peptide chemisch synthetisiert, gereinigt, mittels Massenspektrometrie und Reversed Phase HPLC qualitätskontrolliert und in Aliquots lyophilisiert (Firma JERINI AG, Berlin, Deutschland). Die Aminosäuresequenzen der Peptide lauten:

| | | |
|---|---|---|
| Peptid PCEN17: | CEFEGQPVDFVDPNKQN | SEQ ID NO:7 |
| Peptid PCVD14: | CVPWNHDFTKMEYD | SEQ ID NO:8 |

Rekombinantes Standardmaterial wurde von der Fima InVivo GmbH (Hennigsdorf, Deutschland) bezogen. Es handelte sich um einen kruden Zellextrakt eines E. coli-Stamms, der den rekombinanten N-terminalen Bereich von humaner CPS 1 (Pos. 1-640 aus SEQ ID NO:6), ergänzt um einen N-terminalen Streptag, exprimierte. Dem Extrakt wurde eine arbiträre Konzentration an CPS 1 zugeschrieben.

### 4.1.2. Konjugation und Immunisierung

Mittels MBS (m-Maleimidobenzoyl-N-hydroxysuccinimid Ester) wurden die o.g. Peptide PCEN17 und PCVD14 an das Trägerprotein KLH (Keyhole limpet hemocyanin) konjugiert (s. Arbeitsanleitung "NHS-Esters-Maleimide Crosslinkers" Firma PIERCE, Rockford, IL, USA). Mit diesen Konjugaten wurden Schafe nach folgendem Schema immunisiert: Jedes Schaf erhielt initial 100 µg Konjugat (Massenangabe bezogen auf den Peptid-Anteil des Konjugats) und anschließend 4-wöchentlich je 50 µg Konjugat (Massenangabe bezogen auf den Peptid-Anteil des Konjugats). Beginnend mit dem vierten Monat nach Beginn der Immunisierung wurden 4-wöchentlich je Schaf 700 ml Blut abgenommen und daraus durch Zentrifugation Antiserum gewonnen. Konjugationen, Immunisierungen und Gewinnung von Antiseren wurden von der Firma MicroPharm, Carmarthenshire, UK, durchgeführt.

### 4.1.3. Reinigung der Antikörper

In einem 1-Schritt Verfahren wurden aus den Antiseren, die beginnend mit dem vierten Monat nach der Immuniserung gewonnen worden waren, die Peptid-spezifischen Antikörper präpariert.

Dazu wurden zunächst die Peptide PCEN17 und PCVD14 an SulfoLink Gel gekoppelt (s. Arbeitsanleitung "SulfoLink Kit" Firma PIERCE, Rockford, IL, USA). Dabei wurden zur Kopplung je 5 mg Peptid pro 5 ml Gel angeboten.

Die Affinitätsreinigung von Peptid-spezifischen Antikörpern aus Schaf Antiseren gegen beide Peptide wurde wie folgt durchgeführt:

Die Peptid-Säulen wurden zunächst drei mal im Wechsel mit je 10 ml Elutionspuffer (50 mM Citronensäure, pH 2.2) und Bindungspuffer (100 mM Natriumphosphat, 0.1% Tween, pH 6.8) gewaschen. 100 ml der Antiseren wurden über 0,2 µm filtriert und mit dem vorhandenen Säulenmaterial versetzt. Dazu wurde das Gel quantitativ mit 10 ml Bindungspuffer aus der Säule gespült. Die Inkubation erfolgte über Nacht bei Raumtemperatur unter Schwenken. Die Ansätze wurden quantitativ in Leersäulen (NAP 25, Pharmacia, entleert) überführt. Die Durchläufe wurden verworfen. Anschließend wurde mit 250 ml Bindungspuffer proteinfrei (Proteingehalt des Wascheluats < 0,02 A280 nm) gewaschen. Auf die gewaschene Säulen wurde Elutionspuffer gegeben, und es wurden Fraktionen ä 1 ml gesammelt. Von jeder Fraktion wurde der Proteingehalt mittels der BCA-Methode (s. Arbeitsanleitung Firma PIERCE, Rockford, IL, USA) bestimmt. Fraktionen mit Proteinkonzentrationen > 0.8 mg/ml wurden gepoolt. Nach Proteinbestimmung der Pools mittels der BCA-Methode ergaben sich Ausbeuten von 27 mg für den anti- PCEN17 Antikörper und 33 mg für den anti- PCVD14 Antikörper.

### 4.1.4. Markierung

Über eine NAP-5 Gelfiltrationssäule (Pharmacia) wurden 500 µl des gereinigten anti- PCEN17 Antikörpers (s.o.) in 1 ml 100 mM Kalium-Phosphatpuffer (pH 8,0) nach Arbeitsanleitung umgepuffert. Die Proteinkonzentationsbestimmung der Antikörperlösung ergab einen Wert von 1,5 mg/ml.

Zur Chemilumineszenzmarkierung des Antikörpers wurden 67 µl der Antikörperlösung mit 10 µl MA70-Akridinium-NHS-Ester (1 mg/ml; Firma HOECHST Behring) versetzt und 15 Minuten bei Raumtemperatur inkubiert. Dann wurden 423 µl 1 M Glycin zugesetzt und weitere 10 Minuten inkubiert. Anschließend wurde der Markierungsansatz über eine NAP-5 Gelfiltrationssäule (Pharmacia) in 1 ml Laufmittel A (50 mM Kaliumphosphat, 100 mM NaCl, pH 7.4) nach Arbeitsanleitung umgepuffert und dabei von niedermolekularen Bestandteilen befreit. Zur Abtrennung letzter Reste nicht an Antikörper gebundenen Labels wurde eine Gelfiltrations-HPLC durchgeführt (Säule: Waters Protein Pak SW300). Die Probe wurde aufgetragen und bei einer Flußrate von 1 ml/min mit Laufmittel A chromatographiert. Mit einem Duchflußphotometer wurden die Wellenlängen 280 nm und 368 nm gemessen. Das Absorptionsverhältnis 368 nm/280 nm als Maß für den Markierungsgrad des Antikörpers betrug am Peak 0.10. Die monomeren Antikörper enthaltenden Fraktionen (Retentionszeit 8-10 min) wurden gesammelt und in 3 ml 100 mM Natriumphosphat, 150 mM NaCl, 5% Bovines Serum Albumin, 0.1% Natrium Azid, pH 7.4, gesammelt.

### 4.1.5. Kopplung

Bestrahlte 5 ml Polystyrolröhrchen (Firma Greiner) wurden mit gereinigtem anti-PCVD14 Antikörper wie folgt beschichtet: Der Antikörper wurde in 50 mM Tris, 100 mM NaCl, pH 7,8 zu einer Konzentration von 6.6 µg/ml verdünnt. In jedes Röhrchen wurden 300 µl dieser Lösung pipettiert. Die Röhrchen wurden 20 Stunden bei 22°C inkubiert. Die Lösung wurde abgesaugt. Dann wurde jedes Röhrchen mit 4.2 ml 10 mM Natriumphosphat, 2% Karion FP, 0.3% Bovines Serum Albumin, pH 6.5 befüllt. Nach 20 Stunden wurde die Lösung abgesaugt. Schließlich wurden die Röhrchen in einem Vakuumtrockner getrocknet.

### 4.2. Durchführung und Auswertung des Immunoassays

### 4.2.1. Assaygestaltung

Es wurde ein Assaypuffer folgender Zusammensetzung hergestellt:

100 mM Natriumphosphat, 150 mM NaCl, 5% Bovines Serum Albumin, 0.1% unspez. Schaf IgG, 0.1% Natrium Azid, pH 7.4 Als Standardmaterial diente in E. coli exprimiertes rekombinantes humanes CPS 1 in Form eines kruden E. coli-Extrakts, enthaltend das gesamte lösliche intrazelluläre Protein. Dieser Extrakt wurde seriell in Pferdenormalserum (Firma SIGMA) verdünnt: Den so hergestellten Standards wurden arbiträre Konzentrationen gemäß ihrer Verdünnung zugeschrieben.

### 4.2.2. Messung von EDTA-Plasmen von augenscheinlich Gesunden und von Patienten mit Sepsis.

In die o.g. Teströhrchen wurden je 50 µl Standard bzw. Probe sowie 200 µl Assaypuffer pipettiert. Es wurde 18 Stunden bei 22°C unter Schütteln inkubiert. Dann wurde 4 x mit je 1 ml Waschlösung (0.1% Tween 20) pro Röhrchen gewaschen. Dann wurde in jedes Röhrchen 200 µl Assaypuffer, enthaltend 0.5 Millionen RLU des MA70-markierten Tracer-Antikörpers, pipettiert. Es wurde zwei Stunden bei 22°C unter Schütteln inkubiert. Dann wurde 4 x mit je 1 ml Waschlösung (0.1% Tween 20) pro Röhrchen gewaschen, abtropfen gelassen und die am Röhrchen gebundene Chemilumineszenz in einem Luminometer (Firma BERTHOLD, LB952T; Basisreagenzien BRAHMS AG) vermessen.

Unter Verwendung der Software MultiCalc (Spline Fit) wurde die Konzentration an CPS 1 Immunreaktivität abgelesen. Die Ergebnisse sind in Figur 2 gezeigt. Es ist eine eindeutige Unterscheidung von Gesunden und Sepsispatienten zu erkennen.

### 5. Western-Blot Analysen von Plasmen

Zur näheren molekularen Charakterisierung der CPS 1 Immunreaktivität in Sepsisplasmen wurden Proben solcher Plasmen mittels Western-Blot analysiert:

### 5.1. Gelherstellung

Es wurde ein 7,5%iges SDS-Trenngel für eine PROTEAN II xi Cell (Fa. BIO-RAD) gemäß Anleitung Fa. Bio-Rad gegossen:
11.25 ml 1 M Tris pH 8,8
+ 7,5 ml 30% Acrylamid/ Bisacrylamid (29:1), Fa. Biorad
+ 10,79 ml Milli-Q-Wasser
+ 300 µl 10% SDS
+ 150 µl 10% APS
+ 15 µl TEMED

Nach Überschichtung mit Wasser und Polymerisation wurde ein 5%iges SDS-Sammelgel wie folgt gegossen:
1.25 ml 1 M Tris pH 6,8
+ 1.33 ml 30% Acrylamid/ Bisacrylamid (29:1), Fa. Biorad
+ 7.26 ml Milli-Q-Wasser
+ 100 µl 10% SDS
+ 50 µl 10% APS
+ 10 µl TEMED

5 ml Sammelgellösung wurden auf das Trenngel pipettiert, der Kamm eingesteckt und polymerisieren gelassen.

### 5.2. Gelelektrophorese

Je 5 µl von EDTA-Plasmaproben von drei gesunden Kontrollpersonen sowie von drei Sepsispatienten wurden mit 20 µl PBS, 2.5 µl Glycerin und 5 µl Cracking buffer (120 mM Tris/HCl, pH 6.4, 2% SDS, 20% Glycerin, 20% β-Mercaptoethanol, 0.002% Bromphenolblau) versetzt und 10 min bei 90°C inkubiert, dann aufgetragen. Als Molekulargewichtsmarker wurden 10 µl Rainbow Marker RPN 756 (Fa. Pharmacia) aufgetragen.

Als Kammer wurde eine PROTEAN II xi Cell (Fa. BIO-RAD) verwendet. Elektrophoresepuffer war: 25 mM Tris/HCl, 90 mM Glycin, 0.1% SDS, pH 8.6. Die Elektrophoresebedingungen waren: 45 min bei 46V/16 mA, 30 min bei 120 V/50 mA, 150 min bei 150V/56 mA, 90 min bei 190 V/45 mA.

### 5.3. Blot

Als Blot-Puffer wurde verwendet: 25 mM Tris, 192 mM Glycin, 1% SDS, 20 % Methanol, pH 8,3. Blotfolie war Nitrozelluloseblotfolie Protran BA83, 13 x 13 cm (Fa. Schleicher & Schuell). Die Blot-Apparatur war ein Semi-Dry-Blotter (Modell Pegasus von Fa. Phase).

Das Gel wurde 10 min in Blot-Putter inkubiert und auf die Blotfolie gelegt und mit mehreren Lagen Whatman 3MM Chromatographie Papier (in Blot-Puffer getränkt) beschichtet. Dann wurde geblottet (0,8 mA/cm2 Gelfläche, 70 min).

### 5.4. Immunreaktion:

Die Blotfolie wurde in 150 ml PBS-Tween-Protein Lösung (PBS, 0.3% Tween, 1.5 % BSA, 50 µg/ml unspez. Maus IgG) über Nacht bei 4 °C unter Schütteln abgesättigt. In die Lösung wurden dann je 30 µl Schaf anti- PCEN17 bzw. anti-PCVD14 Antiserum (Herstellung der Antiseren s.o.) gegeben, und es wurde 1 h bei Raumtemperatur unter Schütteln inkubiert. Die Lösung wurde dekantiert, und die Blotfolie wurde 4 x 10 min in je 300ml PBS-Tween-Protein Lösung unter Schütteln gewaschen. Dann wurde der sekundäre Antikörper zugegeben: 30 µl monoklonaler Maus anti-Schaf IgG-alkalische Phosphatase Konjugat (Fa. Sigma, A8062), verdünnt in 150 ml PBS-Tween-Protein Lösung. Es wurde 90 min unter Schütteln bei Raumtemperatur inkubiert. Dann wurde dekantiert und 10 min mit 150 ml PBS-Tween-Protein Lösung unter Schütteln gewaschen. Dann wurde dekantiert und 2 x 10 min mit 150 ml Waschpuffer (100 mM Tris/HCl, pH 7.5, 150 mM NaCl) unter Schütteln gewaschen.

Es wurde Substrat-Lösung wie folgt hergestellt: 100 ml Entwicklungspuffer (100 mM Tris/HCl, pH 9.5, 100 mM NaCl, 50 mM MgCl2) + 350 µl einer Lösung von 50 mg BCIP (5-Bromo-4 Chloro-3 Indolyl Phosphate, Fa. Sigma) pro ml 100% Dimethylformamid, + 450 µl einer Lösung von 100 mg NBT (Nitro blue tetrazolium, Fa. Sigma) pro ml 70% Dimethylformamid.

Die Substratlösung wurde auf die Blotfolie gegeben. Nach 5 Minuten wurde die Farbreaktion durch Waschen der Blotfolie in Wasser beendet. Die Ergebnisse sind in Figur 3 (Panel A) gezeigt.

In einem parallelen Ansatz waren zu der Lösung mit den primären Antiseren (Schaf anti- PCEN17 bzw. anti-PCVD14 Antiserum) die entsprechenden immunogenen Peptide PCEN17 und PCVD14 in einer Endkonzentration von je 2 µg/ml zugegeben und 30 min vorinkubiert worden. Die Ergebnisse sind in Figur 3 (Panel B) gezeigt, wobei auf die Legende zu dieser Figur 3 ausdrücklich Bezug genommen wird.

### 6. Gelfiltrations-HPLC von Sepsis-Plasma

Zur Ermittlung des apparenten Molekulargewichts der CPS 1 Immunreaktivität aus Sepsis-Plasma in Lösung wurde ein solches Plasma über eine Gelfiltrations-HPLC fraktioniert und die CPS1 Immunreaktivität in den Fraktionen gemessen. Kalibriert wurde die Säule durch eine separate Chromatographie von Standards (Bio-Rad-Standard: Cat.No. 151-1901). Es wurde eine Bio-Sil SEC-400 Säule (7,8x300mm Ser.No.415949) der Firma Bio-Rad verwendet. Laufmittel war 300 mM Kaliumphosphat, 0.1% NaN₃, pH 7.0. Vom Sepsis-Plasma wurden 100 µl chromatographiert, es wurden Fraktionen ä 1 ml gesammelt, und je 50 µl davon im Immunoassay vermessen (Durchführung s.o.). Die erhaltenen Ergebnisse (Reaktivität/Fraktion) sind in Figur 4 dargestellt, wobei auf die Legende zu dieser Figur 4 ausdrücklich Bezug genommen wird.

Die Ergebnisse der Messungen der CPS 1-Immunreaktivität in Humanplasmen bzw. der Untersuchungen zur Spezies, die für die beobachtete Immunreaktivität verantwortlich war, lassen sich wie folgt zusammenfassen:

Mittels des beschriebenen Sandwichimmunoassays wurde gezeigt, daß Plasmen von Sepsispatienten stark erhöhte Konzentrationen von CPS 1 Immunreaktivität aufweisen, während in Plasmen von Gesunden CPS 1 nicht zu detektieren war (Fig. 2).

Bei der in Sepsisplasmen zirkulierenden CPS 1-Immunreaktivität handelt es sich offenbar im wesentlichen um das intakte Enzym CPS 1 bzw. eine Form davon mit erhöhter Löslichkeit.

Drei untersuchte Sepsisplasmen zeigten im Western-Blot eine spezifische CPS-Bande bei ca. 150 kDa (Fig. 3). Das entspricht etwa dem für das intakte CPS 1, auf Basis der bekannten Aminosäuresequenz, errechneten Molekulargewicht von rund 160 kDa.

Die Gelfiltrations-HPLC zeigte, daß die CPS 1-Immunreaktivität des untersuchten Sepsis-Plasmas in Lösung ein Molekulargewicht von ca. 200 kDa (+/- 50 kDa) aufweist (Fig. 4).

Die Bemessung von CPS 1 in humanem Serum/Plasma wurde bislang nicht beschrieben, weder für Patienten mit Sepsis noch für andere Krankheitsbilder. Lediglich in einem experimentellen Rattenmodell für akute Hepatitis wurde CPS 1 in Plasma gemessen (s.o. Ozaki et al., 1996). Die Verhältnisse in der Ratte sind aber offenbar nicht mit dem Menschen vergleichbar, denn in der genannten Veröffentlichung wurden bereits für gesunde Tiere CPS-Konzentrationen von 1-2 µg/ml detektiert, während die hierin beschriebenen Messungen von humanen Plasmen Gesunder durch die Anmelderin Werte unterhalb der Nachweisgrenze (abgeschätzt ca. 0.5 ng/ml) erbrachten.

Überraschenderweise zeigte sich für Sepsispatienten eine massive Erhöhung der CPS 1 Immunreaktivität in Plasma. Es ist bekannt, daß bei der Sepsis eine Schädigung der Mitochondrien eintritt (Crouser ED et al., Endotoxin-induced mitochondrial damage correlates with impaired respiratory activity; Crit Care Med. 2002 Feb; 30(2):276-84). Eine derartige Schädigung in Verbindung mit Nekrose oder Apoptose könnte Ursache des Übertritts von CPS 1 aus der mitochondrialen Matrix in die Blutzirkulation sein. Da CPS 1 nahezu ausschließlich in der Leber exprimiert wird und dort einen erheblichen Teil des gesamten löslichen Proteins ausmacht, könnte die Bemessung von CPS 1 in besonderer Weise geeignet sein, um Schädigungen der Leber bei der schweren Sepsis oder anderen Zusammenhängen, z.B. im Rahmen eines Multiorganversagens, anzuzeigen.

## Patentansprüche

1. *In vitro* Verfahren zur differentialdiagnostischen Früherkennung und Erkennung, für die Verlaufsprognose und die Beurteilung des Schweregrads und zur therapiebegleitenden Verlaufsbeurteilung von Sepsis, **dadurch gekennzeichnet, dass** man in der Zirkulation eines menschlichen Patienten die Anwesenheit und/oder Menge von Carbamoylphosphat Synthetase 1 (CPS 1) bestimmt und aus dem Nachweis und/oder der Menge von CPS 1 Schlüsse hinsichtlich des Vorliegens, des zu erwartenden Verlaufs, des Schweregrads der Sepsis oder des Erfolgs ihrer therapeutischen Behandlung zieht.

2. Verfahren nach Anspruch 1, **dadurch gekennzeichnet, dass** die zur Bestimmung genutzten CPS 1-Sequenzen Sequenzen von Fragmenten aus einem die Aminosäuren 1 bis ca. 630 der vollständigen CPS 1-Sequenz (SEQ ID NO:6) umfassenden N-terminalen Teil der CPS 1 sind.

3. Verfahren nach Anspruch 2 oder 3, **dadurch gekennzeichnet, dass** die bestimmte CPS 1-Immunreaktivität wenigstens teilweise Plasmabestandteilen mit einer gelelektrophoretisch bestimmten molaren Masse von 200 kDa ± 50 kDa und/oder Bestandteilen mit einer molaren Mase von 68 bis 70 kDa ± 3 kDa und isoelektrischen Punkten in einem Bereich von 5,5 bis 6,1 zugeordnet werden kann.

4. Verfahren nach einem der Ansprüche 1 bis 3, **dadurch gekennzeichnet, dass** die die Assayzwecke genutzten CPS 1-Sequenzen wenigstens 2 Aminosäure-Teilsequenzen gemäß SEQ ID NO:1, SEQ ID NO:2, SEQ ID NO:3, SEQ ID NO:4 und/oder SEQ ID NO:5, SEQ ID NO:7 oder SEQ ID NO:8 enthalten.

5. Verfahren nach einem der Ansprüche 1 bis 4, **dadurch gekennzeichnet, dass** es ein immundiagnostisches Bestimmungsverfahren ist.

6. Verfahren nach einem der Ansprüche 1 bis 4, **dadurch gekennzeichnet, dass** die Bestimmung der löslichen CPS 1 als Bestimmung der CPS 1-Enzymaktivität in Blut, Blutplasma oder Serum erfolgt.

7. Verfahren nach einem der Ansprüche 1 bis 6, **dadurch gekennzeichnet, dass** es für die Sepsisdiagnostik im Rahmen einer Multiparameter-Bestimmung durchgeführt wird, bei der gleichzeitig mindestens ein weiterer Sepsisparameter bestimmt wird und ein Messergebnis in Form eines Satzes von mindestens zwei Messgrößen gewonnen wird, der zur Sepsis-Feindiagnostik ausgewertet wird.

8. Verfahren nach Anspruch 7, **dadurch gekennzeichnet, dass** im Rahmen der Multiparameter-Bestimmung neben wenigstens einem CPS 1-Fragment wenigstens ein weiterer Parameter bestimmt wird, der ausgewählt ist aus der Gruppe, die besteht aus Procalcitonin, CA 19-9, CA 125, S100B, S100A-Proteinen, löslichen Cytokeratin-Fragmenten, insbesondere CYFRA 21, TPS und/oder löslichen Cytokeratin-1-Fragmenten (sCY1F), den Peptiden Inflammin, CHP, LASP-1 sowie Peptid-Prohormon-Immunreaktivität und dem C-reaktiven Protein (CRP).

9. Verfahren nach Anspruch 7 oder 8, **dadurch gekennzeichnet, dass** die Multiparameter-Bestimmung als Simultanbestimmung mittels einer Chiptechnologie-Messvorrichtung oder einer immunchromatographischen Messvorrichtung erfolgt.

10. Verfahren nach Anspruch 9, **dadurch gekennzeichnet, dass** die Auswertung des mit der Messvorrichtung gewonnenen komplexen Messergebnisses mit Hilfe eines Computerprogramms erfolgt.

## Claims

1. *In vitro* method for the early differential diagnosis and detection, for the prognosis and the assessment of the severity and for the therapy-accompanying monitoring of sepsis, **characterized in that** the presence and/or amount of carbamoyl phosphate synthetase 1 (CPS 1) in the circulation of a human patient is determined, and conclusions are drawn with respect to the presence, the expected course, the severity of the sepsis or the success of its therapeutic treatment from the detection and/or the amount of CPS 1.

2. Method according to Claim 1, **characterized in that** the CPS 1 sequences used for the determination are sequences of fragments from an N-terminal part of CPS 1, comprising the amino acids 1 to about 630 of the complete CPS 1 sequence (SEQ ID NO:6).

3. Method according to Claim 2 or 3, **characterized in that** the determined CPS 1 immunoreactivity can be at least partially assigned to plasma components having a molar mass, determined by gel electrophoresis, of 200 kDa ± 50 kDa and/or components having a molar mass of from 68 to 70 kDa ± 3 kDa and isoelectric points in a range from 5.5 to 6.1.

4. Method according to any of Claims 1 to 3, **characterized in that** the CPS 1 sequences used for assay purposes contain at least 2 amino acid partial sequences according to SEQ ID NO:1, SEQ ID NO:2, SEQ ID NO:3, SEQ ID NO:4 and/or SEQ ID NO:5, SEQ ID NO:7 or SEQ ID NO:8.

5. Method according to any of Claims 1 to 4, **characterized in that** it is an immunodiagnostic assay method.

6. Method according to any of Claims 1 to 4, **characterized in that** the determination of the soluble CPS 1 is carried out as a determination of the CPS 1 enzyme activity in blood, blood plasma or serum.

7. Method according to any of Claims 1 to 6, **characterized in that** it is carried out for sepsis diagnosis as part of a multiparameter determination, in which at least one further sepsis parameter is simultaneously determined and a measured result in the form of a set of at least two measured quantities is obtained, which is evaluated for the fine diagnosis of sepsis.

8. Method according to Claim 7, **characterized in that**, in addition to at least one CPS 1 fragment, at least one further parameter which is selected from the group consisting of procalcitonin, CA 19-9, CA 125, S100B, S100A proteins, soluble cytokeratin fragments, in particular CYFRA 21, TPS and/or soluble cytokeratin-1 fragments (sCY1F), the peptides inflammin, CHP, LASP-1 and peptide prohormone immunoreactivity and the C-reactive protein (CRP) is determined as part of the multiparameter determination.

9. Method according to Claim 7 or 8, **characterized in that** the multiparameter determination is carried out as a simultaneous determination by means of a chip technology measuring device or of an immunochromatographic measuring device.

10. Method according to Claim 9, **characterized in that** the evaluation of the complex measured result obtained using the measuring device is effected with the aid of a computer program.

## Revendications

1. Procédé in vitro de détection précoce et de détection en diagnostic différentiel, pour le pronostic de l'évolution et l'évaluation du degré de gravité ainsi que pour l'évaluation de l'évolution d'une thérapie de la septicémie, **caractérisé en ce que** l'on détermine dans la circulation d'un patient humain la présence et/ou la quantité de carbamoylphosphate synthétase 1 (CPS 1) et qu'à partir de la détection et/ou de la quantité de CPS 1, on tire des conclusions sur la présence, l'évolution attendue, le degré de gravité de la septicémie ou le résultat de son traitement thérapeutique.

2. Procédé selon la revendication 1, **caractérisé en ce que** les séquences de CPS 1 utilisées pour la détermination sont des séquences de fragments d'une partie de la terminaison N de la CPS 1 qui comprend les acides aminés 1 à environ 630 de la séquence complète de la CPS 1 (SEQ ID NO:6).

3. Procédé selon les revendications 1 ou 2, **caractérisé en ce que** l'immunoréactivité de la CPS 1 déterminée peut être associée à au moins certains composants du plasma dont la masse moléculaire déterminée par électrophorèse sur gel est de 200 kDa ± 50 kDa et/ou à certains composants dont la masse moléculaire est 68 à 70 kDa ± 3 kDa et dont le point isoélectrique est compris dans la plage de 5,5 à 6,1.

4. Procédé selon l'une des revendications 1 à 3, **caractérisé en ce que** les séquences de CPS 1 utilisées pour la détermination contiennent au moins 2 parties de séquences d'acides aminés de SEQ ID NO:1, SEQ ID NO:2, SEQ ID NO:3, SEQ ID NO:4 et/ou SEQ ID NO:5, SEQ ID NO:7 ou SEQ ID NO:8.

5. Procédé selon l'une des revendications 1 à 4, **caractérisé en ce qu'**il s'agit d'un procédé de détermination immunodiagnostique.

6. Procédé selon l'une des revendications 1 à 4, **caractérisé en ce que** la détermination de la CPS 1 soluble s'effectue par détermination de son activité enzymatique dans le sang, dans le plasma sanguin ou dans le sérum.

7. Procédé selon l'une des revendications 1 à 6, **caractérisé en ce qu'**il est exécuté pour le diagnostic de la septicémie dans le cadre d'une détermination de plusieurs paramètres dans laquelle on détermine simultanément au moins un autre paramètre de la septicémie et **en ce qu'**il fournit un résultat de mesure qui présente la forme d'un ensemble d'au moins deux grandeurs de mesure qui sont évaluées pour le diagnostic fin de la septicémie.

8. Procédé selon la revendication 7, **caractérisé en ce que** dans le cadre de la détermination de plusieurs paramètres, en plus d'au moins un fragment de CPS 1, on détermine au moins un autre paramètre sélectionné dans l'ensemble constitué de la procalcitonine, des protéines CA 19-9, CA 125, S100B, S100A, des fragments solubles de cytokératine et en particulier du CYFRA 21, de la TPS et/ou des fragments solubles de cytokératine-1 (sCY1F), des peptides inflammine, CHP, LASP-1 ainsi que de l'immunoréactivité de prohormones peptidiques et de la protéine C-réactive.

9. Procédé selon les revendications 7 ou 8, **caractérisé en ce que** la détermination de plusieurs paramètres s'effectue comme détermination simultanée au moyen d'un dispositif de mesure qui recourt à la technologie des puces ou d'un dispositif de mesure par immunochromatographie.

10. Procédé selon la revendication 9, **caractérisé en ce que** l'évaluation du résultat complexe de mesure obtenu avec le dispositif de mesure s'effectue à l'aide d'un programme informatique.


### SEQUENCE LISTING

<110> B.R.A.H.M.S Aktiengesellschaft
<120> Verwendungen der Carbamoylphosphat Synthetase 1 (CPS 1) und ihrer Fragmente für die Diagnose von Entzündungserkrankungen und Sepsis
<130> 3695PCT AS
<140>
   <141>
<150> 02008841.5 EP
   <151> 2002-04-19
<160> 8
<170> PatentIn Ver. 2.1
<210> 1
   <211> 11
   <212> PRT
   <213> Primat (Pavian)
<400> 1
   Gly Gln Asn Gln Pro Val Leu Asn Ile Thr Asn
   1 5 10
<210> 2
   <211> 7
   <212> PRT
   <213> Primat (Pavian)
<400> 2
   Asn Gln Pro Val Leu Asn Ile
   1 5
<210> 3
   <211> 13
   <212> PRT
   <213> Primat (Pavian)
<400> 3
   Ala Gln Thr Ala His Ile Val Leu Glu Asp Gly Thr Lys
   1 5 10
<210> 4
   <211> 4
   <212> PRT
   <213> Primat (Pavian)
<400> 4
   Thr Ala His Ile
   1
<210> 5
   <211> 12
   <212> PRT
   <213> Primat (Pavian)
<400> 5
   Ala Phe Ala Met Thr Asn Gln Ile Leu Val Glu Lys
   1 5 10
<210> 6
   <211> 1500
   <212> PRT
   <213> Homo sapiens
<400> 6
<210> 7
   <211> 17
   <212> PRT
   <213> Artificial Sequence
<220>
   <223> Description of Artificial Sequence: Synthetic Peptide
<400> 7
   Cys Glu Phe Glu Gly Gln Pro Val Asp Phe Val Asp Pro Asn Lys Gln
   1 5 10 15
   Asn
<210> 8
   <211> 14
   <212> PRT
   <213> Artificial Sequence
<220>
   <223> Description of Artificial Sequence: Synthetic Peptide
<400> 8
   Cys Val Pro Trp Asn His Asp Phe Thr Lys Met Glu Tyr Asp
   1 5 10